# EUROPEAN PATENT SPECIFICATION

(11) **EP 3 851 896 B1**
(45) Date of publication and mention of the grant of the patent: **04.03.2026**
(21) Application number: 20152639.9
(22) Date of filing: 20.01.2020
(51) Int. Cl.: A61B 34/20, A61B 90/25, A61B 90/00, G02B 21/00, A61B 34/00, A61B 34/30, A61B 90/50

(54) **APPARATUSES, METHODS AND COMPUTER PROGRAMS FOR CONTROLLING A MICROSCOPE SYSTEM**
VORRICHTUNGEN, VERFAHREN UND COMPUTERPROGRAMME ZUR STEUERUNG EINES MIKROSKOPSYSTEMS
APPAREILS, PROCÉDÉS ET PROGRAMMES D'ORDINATEUR POUR COMMANDER UN SYSTÈME DE MICROSCOPE

(43) Date of publication of application: 21.07.2021
(73) Proprietor: Leica Instruments (Singapore) Pte. Ltd., 608924 Singapore (SG)
(72) Inventor: Themelis, George, 88131 Lindau (DE)
(74) Representative: 2SPL Patentanwälte PartG mbB

(56) References cited:
- EP-B1- 3 125 737

## Description

### Technical field

Examples relate to apparatuses, methods and computer programs for controlling a microscope system, and to a corresponding microscope system.

### Background

Surgical microscopes systems are often designed so that an optical carrier (i.e. the surgical microscope) can be moved as easy and smoothly as possible, ideally giving the sense of lack of gravity. In some cases, a robotic adjustment system, such as a robotic arm, is used to move the surgical microscope. The movement is often performed using knobs and buttons, which are used to individually control a position of the surgical microscope, an angle of the surgical microscope and other functionality, such as a zoom level, illumination etc. Due to the many factors that can be adjusted in order to obtain the desired field of view of the surgical microscope, controlling the robotic adjustment system may take up time and concentration of the surgeon. US patent 5,345,087 shows an optical guide system for spatially positioning a surgical microscope, which provides a contactless guidance of the instrument. In the patent, light sources are attached to the head of the surgeon and are sensed to determine the orientation of the head of the surgeon, and correspondingly control a motorized adjustment mechanism. The head is thereby, in an initialization phase, brought to a pre-defined position, and the movement of the head is tracked relative to this pre-defined position. This provides a basic contactless control of the motorized adjustment mechanism that does not consider the mechanics that humans employ when taking different visual perspectives.

European patent EP 3 125 737 B1 relates to a surgical control device and imaging control system. In said patent, a medical observation system comprises a video microscope, a head mount display and an electric arm. Using a gyroscope in the head mount display, or via an external camera, a position and motion of a head of the user is determined, and a position of the video microscope is changed via the electric arm.

There may be a desire for an improved microscope system, that provides an improved control of the robotic adjustment system.

### Summary

Embodiments of the present disclosure are based on the finding that other systems merely provide a purely mechanical movement equivalent to the head movement of the surgeon, instead of determining the perspective that the surgeon desires to gain when performing a head movement. In embodiments, the angular orientation of the head of the user is determined relative to a point - relative to a display, or relative to the surgical site, and this angular orientation is used to determine which perspective the operator is trying to take vis-à-vis the point, and how the robotic adjustment system can be adjusted to achieve this desired change in perspective. Embodiments may thus use the head movements, gestures and expressions to control the microscope, providing head tracking for robot control in a intuitive manner. One embodiment is the adjustment of a microscope's alignment by robot which follows the head of the user.

Embodiments of the present disclosure provide an apparatus for controlling a microscope system. The apparatus comprises an interface for communicating with a camera module. The camera module is suitable for providing camera image data of a head of a user of the microscope system. The apparatus comprises a processing module configured to obtain the camera image data from the camera module via the interface. The processing module is configured to process the camera image data to determine information on an angular orientation of the head of the user relative to a display of the microscope system. The processing module is configured to provide a control signal for a robotic adjustment system of the microscope system based on the information on the angular orientation of the head of the user. By determining the angular orientation of the head of the user relative to a display of the microscope system, changes of the orientation of the head relative to the display can be translated into a change of perspective of the microscope system in a comprehensible manner, improving a control of the microscope system by the user/surgeon of the microscope system.

In at least some embodiments, the processing module is configured to determine an angle of view of the user of the microscope system towards the display based on the camera image data. The processing module may be configured to provide the control signal based on the angle of view of the user. The angle of view may be used to adjust the robotic arm such, that the microscope provides a field of view that is derived from the angle of view.

For example, the processing module may be configured to determine the angle of view of the user based on the angular orientation of the head of the user. The angular orientation may provide a coarse-grained determination of angle of view.

In some embodiments, the processing module is configured to perform eye tracking on one or both eyes of the user. The processing module may be configured to determine the angle of view of the user based on the angular orientation of the head of the user and based on the eye tracking. This may provide a more fine-grained determination of the angle of view.

The processing module may be configured to determine the information on the angular orientation using image analysis on the camera image data. This may enable a determination of the angular orientation without the user having to wear additional tracking markers.

In some embodiments, the display of the microscope system is an auxiliary display that is arranged in proximity of an eyepiece of a microscope of the microscope system. This may enable the surgeon an in-situ adjustment of the robotic arm, without having to leave the surgical field.

Alternatively, the display of the microscope system may be an auxiliary display that is arranged at a base unit of the microscope system. This may enable the use of larger displays, which may be used in preparation of the surgical procedure or by additional surgical staff.

In some embodiments, the display of the microscope system may be an ocular display of an eyepiece of a microscope of the microscope system. Again, this may enable the surgeon an in-situ adjustment of the robotic arm, without having to leave the surgical site.

In various embodiments, the processing module is configured to determine a fixation of the user on a point based on the camera image data. The processing module may be configured to determine the control signal for the robotic adjustment system such that a (central) point of view of a microscope of the microscope system remains fixed on the point. The (central) point (e.g. the point, on which the fixation is focused) may serve as a visual anchor in the adjustment of the robotic arm.

The processing module may be configured to determine information on a position of the head of the user and information on an angle of the head of the user based on the camera image. The processing module may be configured to determine the information on the angular orientation of the head of the user based on the information on the position of the head of the user and based on the information on the angle of the head of the user. Both the position and the angle (e.g. absolute or relative to the display) may be factors in the determination of the angular orientation, as the angular orientation is defined relative to the display.

For example, the processing module may be configured to generate the control signal such that the robotic adjustment system is triggered to pan a field of view of a microscope of the microscope system if the position of the head of the user changes without a corresponding change in the angle of the head of the user. This may provide a natural translation between the movement of the position of the head and the orientation of the robotic arm.

Additionally or alternatively, the processing module may be configured to generate the control signal such that the robotic adjustment system is triggered to adjust an angle of view of a microscope of the microscope system if the position of the head of the user changes with a corresponding change in the angle of the head of the user. This may provide a comprehensible translation between a change of the angle of the head and the orientation of the robotic arm.

In some embodiments, the interface is suitable for obtaining a trigger signal from an input device of the microscope system. The processing module may be configured to provide the control signal in response to the trigger signal of the input device. Thus, the control signal might only be provided if explicitly triggered by the user, avoiding an undesired adjustment of the robotic arm.

For example, the control signal may be configured to adjust at least one of a position in space of a microscope of the microscope system, an observation angle of the microscope, a zoom of the microscope, a working distance of the microscope, and an illumination of the microscope. For example, multiple elements of the above group may be adjusted at the same time, providing a coordinated adjustment of the various properties of the microscope.

Embodiments further provide a further apparatus for controlling a microscope system. The apparatus comprises an interface for communicating with a camera module. The camera module is suitable for providing camera image data of a head of a user of the microscope system. The apparatus comprises a processing module configured to obtain the camera image data from the camera module via the interface. The processing module is configured to process the camera image data to determine information on an angular orientation of the head of the user and to determine a fixation of the user on a point. The processing module is configured to provide a control signal for a robotic adjustment system of the microscope system based on the information on the angular orientation of the head of the user such that a (central) point of view of a microscope of the microscope system remains fixed on the point. By determining the angular orientation of the head of the user, changes of the orientation of the head relative to the display can be translated into a change of perspective of the microscope system in an organic manner, improving an interaction between the surgeon and the microscope system. The fixation on the user on the point may provide a visual anchor for the adjustment of the robotic arm.

For example, the processing module may be configured to determine the information on the angular orientation of the head of the user of the microscope relative to a display of the microscope system. In other words, the display may be used as a point of reference for determining the angular orientation, providing the surgeon with a reference in the adjustment of the robotic arm.

Alternatively, the microscope system may be a surgical microscope system. The processing module may be configured to determine the information on the angular orientation of the head of the user of the microscope relative to a surgical site being observed by a surgical microscope of the surgical microscope system. In this case, the surgeon may control the robotic arm by adjusting their angular orientation towards the surgical site, which is then translated to provide an equivalent orientation of the microscope.

Embodiments of the present disclosure further provide a microscope system comprising the one or both of the above apparatuses.

Embodiments of the present disclosure further provide a method for controlling a microscope system. The method comprises obtaining camera image data of a head of a user of a head of a user of the microscope system from a camera module of the microscope system. The method comprises processing the camera image data to determine information on an angular orientation of the head of the user relative to a display of the microscope system. The method comprises providing a control signal for a robotic adjustment system of the microscope system based on the information on the angular orientation of the head of the user.

Embodiments of the present disclosure further provide a method for controlling a microscope system. The method comprises obtaining camera image data of a head of a user of a head of a user of the microscope system from a camera module of the microscope system. The method comprises processing the camera image data to determine information on an angular orientation of the head of the user and to determine a fixation of the user on a point. The method comprises providing a control signal for a robotic adjustment system of the microscope system based on the information on the angular orientation of the head of the user such that a (central) point of view of a microscope of the microscope system remains fixed on the point.

Embodiments of the present disclosure further provide a computer program with a program code for performing at least one of the methods when the computer program is executed on a processor.

### Short description of the Figures

Some examples of apparatuses and/or methods will be described in the following by way of example only, and with reference to the accompanying figures, in which
- Figs. 1a and 1b: show a block diagrams of embodiments of an apparatus for controlling a microscope system and of a microscope system comprising such an apparatus, respectively;
- Fig. 2: shows a flow chart of an embodiment of a method for controlling a microscope system;
- Fig. 3: shows a flow chart of a further embodiment of a method for controlling a microscope system; and
- Fig. 4: shows a schematic diagram of a system comprising a microscope and a computer system.

### Detailed Description

Various examples will now be described more fully with reference to the accompanying drawings in which some examples are illustrated. In the figures, the thicknesses of lines, layers and/or regions may be exaggerated for clarity.

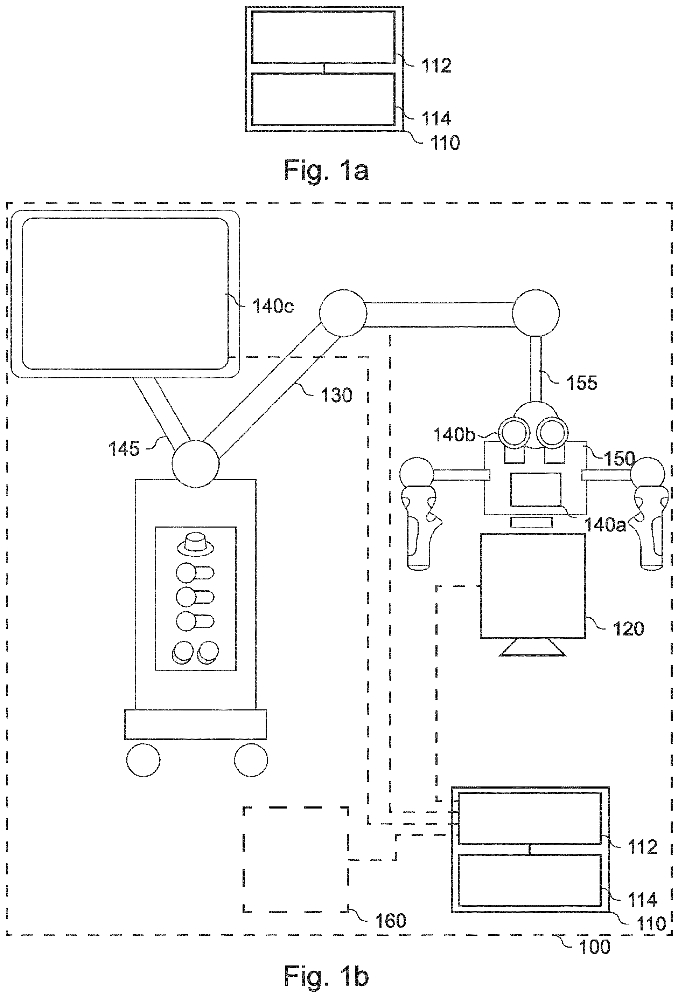

Figs. 1a and 1b show a block diagram of embodiments of an apparatus 110 for controlling a microscope system 100. The apparatus comprises an interface 112 for communicating with a camera module 120. The camera module is suitable for providing camera image data of a head of a user of the microscope system. The apparatus comprises a processing module 114, which is coupled to the interface 112. The processing module 114 is configured to obtain the camera image data from the camera module via the interface.

The camera image data is subsequently processed to determine information on an angular orientation of the head of the user, either relative to a display or relative to a point on which the gaze of the user is fixated (on the display, or on the surgical site). Accordingly, in some embodiments, the processing module 114 is configured to process the camera image data to determine information on an angular orientation of the head of the user relative to a display 140 of the microscope system. In this case, the processing module is configured to provide a control signal for a robotic adjustment system 130 of the microscope system based on the information on the angular orientation of the head of the user. Alternatively or additionally, the processing module 114 may be configured to process the camera image data to determine information on an angular orientation of the head of the user and to determine a fixation of the user on a point. In this case, the processing module is configured to provide the control signal for a robotic adjustment system 130 of the microscope system based on the information on the angular orientation of the head of the user such that a central point of view of a microscope 150 of the microscope system remains fixed on the point.

Fig. 1b further shows the microscope system 100 comprising the apparatus 110, the camera module 120, the robotic adjustment system 130, optionally the display 140, further optionally the microscope 150, and further optionally an input device 160.

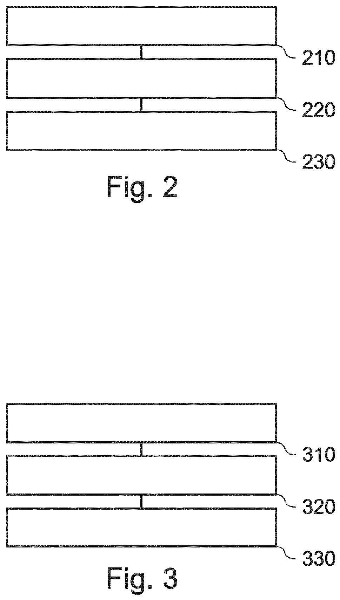

The following description relates to the control apparatus 110 and microscope system 100 of Figs. 1a and 1b, and to the corresponding methods of Figs. 2 and 3.

Embodiments of the present disclosure relate to apparatuses, method and computer programs for controlling a microscope system 100. In general, a microscope is an optical instrument that is suitable for examining objects that are too small to be examined by the human eye (alone). For example, a microscope may provide an optical magnification of an object, such as the sample of organic tissue. A surgical microscope is a microscope that is used during surgery, i.e. a microscope that is suitable for use (by a surgeon) during surgery. Such surgical microscope systems often comprise an arm or a positioning means that is used to position the surgical microscope as desired by the surgeon, e.g. close to the operating space, so the surgical microscope can be used to provide a magnified view of the wound tract or tissue. Such arms or positioning means are usually adapted to provide a wide variety of different positions and/or angles, in order to provide the surgeon with enough space to conduct the surgery, while providing the magnified image of the site of the surgery. Additionally, the arms or positioning means of surgical microscope system are often designed so they allow for easy positioning of the surgical microscope, e.g. using weights and counterweights, using pneumatic systems or using motors to support the movement of the arm or positioning means of the surgical microscope system. As has been laid out above, many microscope systems, such as surgical microscope systems, have a robotic adjustment system, such as a robotic arm, for positioning the microscope of the microscope system relative to a sample to be observed using the microscope system. In other words, the robotic adjustment system may be configured to adjust the arm of the microscope system. For example, the robotic adjustment system may be a robotic arm of the (surgical) microscope system. Such robotic adjustment systems often offer a large number of degrees of freedom regarding the adjustment of the system - in many cases, an angular orientation of the microscope relative to the sample can be adjusted as well as a three-dimensional position of the microscope. Accordingly, adjustments to the robotic arms often are not straightforward. In some systems, the user/operator, e.g. the surgeon, can grab handles that are attached to the microscope and manually reposition of the microscope, aided by the robotic adjustment system, which operate as servo motors in this case, facilitating the movement of the machinery, which is often heavy. In embodiments, however, a control of the robotic adjustment system is chosen that is based on the angular orientation of the head of the user, which is derived by processing of camera image data. In other words, the microscope system may be a surgical microscope system having a robotic adjustment system, such as a robotic arm, which may be controlled based on the angular orientation of the head of the user.

The microscope system comprises the camera module 120, which is suitable for providing camera image data of a head of a user of the microscope system. In other words, the camera module 120 may be configured to provide the camera image data of the head of the user of the microscope system. In general, the camera module may comprise an APS (Active Pixel Sensor) - or a CCD (Charge-Coupled-Device)-based imaging sensor module. For example, in APS-based imaging sensor modules, light is recorded at each pixel using a photodetector and an active amplifier of the pixel. APS-based imaging sensor modules are often based on CMOS (Complementary Metal-Oxide-Semiconductor) or S-CMOS (Scientific CMOS) technology. In CCD-based imaging sensor modules, incoming photons are converted into electron charges at a semiconductor-oxide interface, which are subsequently moved between capacitive bins in the imaging sensor modules by a control circuitry of the sensor imaging module to perform the imaging. Alternatively, or additionally, the camera module may be a depth-sensing camera module or comprise a depth sensor, suitable for providing a depth-sensing camera module image. Accordingly, the camera image data may be a depth-sensing camera image data or comprise a two-dimensional and a depth-sensing component. For example, the camera module may comprise a depth sensor, e.g. a Time of Flight-based depth sensor or a structured light-based depth sensor. The camera image data may comprise two-dimensional camera image data of the head of the user and/or three-dimensional camera image data of the head of the user.

The processing module 114 is configured to process the camera image data to determine the information on an angular orientation of the head of the user, e.g. relative to the display 140 or relative to the surgical site. In general, the angular orientation of the head of the user may comprise an angle of the head relative to a point of reference, e.g. relative to a resting position of the head, relative to the display 140 or relative to the surgical site. Additionally, the angular orientation may comprise a position of the head relative to a coordinate system, which may be relative to the microscope system, relative to the display or relative to the surgical site. In other words, the processing module may be configured to determine information on a position of the head of the user and information on an angle of the head of the user (e.g. relative to a point of reference) based on the camera image. The processing module may be configured to determine the information on the angular orientation of the head of the user based on the information on the position of the head of the user and based on the information on the angle of the head of the user. The information on the angular orientation may comprise numerical values of the angle of the head (relative to the point of reference) and/or of the position of the head.

The processing of the camera image data may be performed using image analysis. In other words, the processing module may be configured to determine the information on the angular orientation using image analysis on the camera image data. For example, the image analysis may be used identify one or more features of the head within the camera image data. For example, the processing module may be configured to determine the angular orientation of the head of the user by determining an outline of the head of the user, by identifying eyes and/or a nose within the (two-dimensional or three-dimensional) camera image data, and by deriving the angular orientation of the head of the user based on the outline of the head and based on the position of the eyes and/or the nose relative to the outline of the head. The image analysis may be based on the (natural) outline of the head. For example, the image analysis might not be based on tracking markers being attached to the head of the user. On the contrary - the processing module may be configured to determine the information on the angular orientation of the head of the user without identifying (artificially added) tracking markers.

In various embodiments, the information on the angular orientation of the head of the user is determined relative to a display 140 of the microscope system. For example, the display 140 may be an electronic output device for presentation of information in visual form. For example, the display 140 may be suitable for and/or configured to electronically display an image of a sample to be observed by the microscope, e.g. an image of a surgical site to be observed by the surgical microscope. The processing module 114 may be configured to provide the image of the sample to be observed to the display 114. In at least some embodiments, the image is based on further camera image data of a further camera (not shown) of the microscope 150. For example, the processing module 114 may be configured to obtain the further camera image data from the further camera and to generate the image for the display 114. For example, the display 140 may be a Liquid-Crystal-Display (LCD)-based display or an Organic Light Emitting Diode (OLED)-based display. In some embodiments, the display may be arranged at or within the eyepiece of the microscope 150 of the microscope system. For example, the display may be arranged above the eyepiece of the microscope, or the display may be integrated within the eyepieces of the microscope. In other words, the display of the microscope system may be an auxiliary display 140a that is arranged in proximity of an eyepiece of a microscope of the microscope system. For example, the display 140a; b may be attached to the microscope 150 of the microscope system. For example, the display of the microscope system may be an ocular display 140b of an eyepiece of a microscope of the microscope system. Alternatively, the display of the microscope system may be an auxiliary display 140c that is arranged at a base unit (e.g. a stand comprising the apparatus 110) of the microscope system. For example, the display 140b may be attached to the base unit of the microscope system via a further arm 145.

As laid out above, in some embodiments, the angular orientation of the head of the user is determined relative to the display 140 or relative to the surgical site being observed by the surgical microscope. For example, this may be achieved by determine the angle of view of the user towards the display or surgical site. In other words, the processing module may be configured to determine an angle of view of the user of the microscope system towards the display (or towards the surgical site) based on the camera image data. For example, the processing module may be configured to derive the angle of view of the user towards the display or surgical site based on the outline of the head and based on the position of the eyes and/or the nose relative to the outline of the head, and based on the position of the head of the user. For example, the processing module may be configured to determine the angle of view of the user based on the angular orientation of the head of the user. For example, the angular orientation of the had of the user may comprise the angle of view of the user towards the display or surgical site, e.g. if an angular component of the angular orientation is determined relative to the display or surgical site as point of reference. In some embodiments, this may suffice to determine an angle of view that is precise enough to perform the adjustment of the robotic adjustment system. In some embodiments, however, the processing module may be configured to perform eye-tracking in order to more precisely learn where the user (i.e. the surgeon) is looking. In other words, the processing module may be configured to perform eye tracking on one or both eyes of the user. For example, the processing module may be configured to perform image processing on the camera image data in order to perform eye tracking on one or both eyes of the user. Alternatively, the image processing may be performed on further camera image data of a dedicated eye tracking camera, e.g. in addition to three-dimensional camera image data that is used to determine the angular orientation. The processing module 114 may be configured to determine the angle of view of the user based on the angular orientation of the head of the user and based on the eye tracking. In other words, the processing module 114 may be configured to refine the determined angle of view of the user based on the eye tracking.

The processing module may be configured to provide the control signal based on the angle of view of the user. For example, the processing module 114 may be configured to translate the angle of view of the user into a corresponding orientation of the microscope 150, and to provide the control signal to affect the corresponding orientation of the microscope 150. For example, the control signal may be provided such, that the robotic adjustment system is affected to arrange the microscope 150 at an angle that is based on the angle of view of the user, e.g. at an angle that corresponds to the angle of view of the user.

When changing the positioning of the microscope, a point (of the sample, or of the surgical site) may be used as "anchor point", which remains fixed while the angle of view or the distance/zoom level is changed, e.g. so the user can concentrate on a point of interest and view it from different angles. Therefore, a fixation of the user on the point may be determined, and the point may serve as anchor point in the adjustment of the microscope (via the robotic adjustment module. In other words, the processing module may be configured to determine a fixation of the user on a point (e.g. a point on the screen, a point of the sample/surgical site shown on the screen, or a point of the surgical site) based on the camera image data. The processing module may be configured to process the camera image data to determine the fixation of the user on the point. For example, the processing module may be configured to determine the fixation of the user on the point using eye tracking, e.g. using the angular orientation of the head of the user in conjunction with eye tracking. The processing module may be configured to determine the control signal for the robotic adjustment system such that a central point of view of the microscope 150 of the microscope system remains fixed on the point. In other words, the processing module may be configured to determine the control signal for the robotic adjustment system such, that the point remains at a similar (e.g. the same position) position within the field of view of the microscope 150.

In general, different approaches may be taken depending on whether the user merely changes the position of the head or whether the user changes the angle of the head. For example, if the user merely changes the position of its head without changing the angle (or with a change of the angle that is lower than a threshold, e.g. lower than 5 degrees), the control signal may be generated such that the field of view of the microscope pans (i.e. scrolls horizontally and/or vertically without changing the angle of view) over the sample/surgical field. In other words, the processing module may be configured to generate the control signal such that the robotic adjustment system is triggered to pan a field of view of the microscope of the microscope system if the position of the head of the user changes without a corresponding change in the angle of the head of the user. If merely the angle of the head is changed, the angle of view may be adjusted without panning. In other words, the processing module may be configured to generate the control signal such that the robotic adjustment system is triggered to adjust an angle of view of the microscope of the microscope system if the position of the head of the user changes with a corresponding change in the angle of the head of the user. Again, the processing module may be configured to determine the control signal for the robotic adjustment system such that a central point of view of the microscope 150 of the microscope system remains fixed on a point within the field of view, e.g. based on a point on which the user is fixated, or based on a central point of the field of view. In other words, the processing module may be configured to determine the control signal for the robotic adjustment system such, that a point, e.g. a point on which the user is fixated, or a central point of the field of view, remains at a similar (e.g. the same position) position within the field of view of the microscope 150. In this case, the change in the angle of view may also result in a panning of the field of view, e.g. based on the point referenced above. Furthermore, in some embodiments, the processing module may be configured to determine information on a position, posture and/or movement of shoulders of the user based on the camera image data, and use the information on the position, posture and/or movement of the shoulders to generate the control signal.

The processing module is configured to provide the control signal for the robotic adjustment system 130 of the microscope system based on the information on the angular orientation of the head of the user. As mentioned above, the processing module 114 may be configured to translate the angle of view of the user into a corresponding orientation of the microscope 150, and to provide the control signal to affect the corresponding orientation of the microscope 150. Various properties of the robotic adjustment system may be adjusted in order to affect the orientation of the microscope 150. For example, the control signal may be configured to adjust at least one of a position in space of the microscope 150 of the microscope system, an observation angle of the microscope, a zoom of the microscope, a working distance of the microscope, and an illumination of the microscope. For example, the control signal may be configured to adjust the position in space, and thereby the working distance, of the microscope 150 by controlling the robotic adjustment system such, that the position in space of the microscope is changed, e.g. by controlling robotic joints of the robotic arm in order to obtain the desired position in space. The control signal may be configured to adjust the observation angle of the microscope by controlling the robotic adjustment system such, that the position in space of the microscope is changed and such that an angle of the microscope towards the sample or surgical site is changed to obtain the observation angle. The control signal may further be configured to control the microscope to change the zoom and/or the illumination of the microscope. Accordingly, the control signal may also be provided to the microscope 150 or to an illumination unit of the microscope system 150.

In some cases, it may be desirable to limit the provision of the control signal to situations, in which the user/surgeon wishes to change the position of the microscope, and to refrain from generating or providing the control signal outside these situations. To achieve this, the user/surgeon may provide a trigger via an input device in order to activate (or deactivate) the generation/provision of the control signal. Accordingly, the interface may be suitable for obtaining a trigger signal from an input device 160 of the microscope system. For example, the input device may be one of a button, a touch-based interface (such as a touchscreen or a capacitive switch), a light-based input device (e.g. by blocking a path between light source and receiver, the input device is actuated), an ultrasound-based input device (e.g. by bringing a hand or object close to the ultrasound-based input device, the input device is actuated), and a voice-activated input device. The processing module may be configured to provide the control signal in response to the trigger signal of the input device. In other words, the processing module may be configured to provide the control signal (only) if triggered by the input device (and/or as long it is triggered by the input device).

The interface 112 may correspond to one or more inputs and/or outputs for receiving and/or transmitting information, which may be in digital (bit) values according to a specified code, within a module, between modules or between modules of different entities. For example, the interface 112 may comprise interface circuitry configured to receive and/or transmit information. In embodiments the processing module 114 may be implemented using one or more processing units, one or more processing devices, any means for processing, such as a processor, a computer or a programmable hardware component being operable with accordingly adapted software. In other words, the described function of the processing module 114 may as well be implemented in software, which is then executed on one or more programmable hardware components. Such hardware components may comprise a general-purpose processor, a Digital Signal Processor (DSP), a micro-controller, etc.

More details and aspects of the microscope system or the apparatus for controlling the microscope system are mentioned in connection with the proposed concept or one or more examples described above or below (e.g. Fig. 2 to 4). The microscope system or the apparatus for controlling the microscope system may comprise one or more additional optional features corresponding to one or more aspects of the proposed concept or one or more examples described above or below.

Fig. 2 shows a flow chart of an embodiment of a (corresponding) method for controlling a microscope system. For example, the microscope system may be implemented similar to the microscope system of Fig. 1b. The method comprises obtaining 210 camera image data of a head of a user of a head of a user of the microscope system from a camera module of the microscope system. The method comprises processing 220 the camera image data to determine information on an angular orientation of the head of the user relative to a display 140 of the microscope system. The method comprises providing 230 a control signal for a robotic adjustment system of the microscope system based on the information on the angular orientation of the head of the user.

As indicated above, features described in connection with the apparatus 110 and the microscope system 100 of Figs. 1a and/or 1b may be likewise applied to the method of Fig. 2.

More details and aspects of the method are mentioned in connection with the proposed concept or one or more examples described above or below (e.g. Fig. 1 or 3 to 4). The method may comprise one or more additional optional features corresponding to one or more aspects of the proposed concept or one or more examples described above or below.

Fig. 3 shows a flow chart of a further embodiment of a (corresponding) method for controlling a microscope system. For example, the microscope system may be implemented similar to the microscope system of Fig. 1b. The method comprises obtaining 310 camera image data of a head of a user of a head of a user of the microscope system from a camera module of the microscope system. The method comprises processing 320 the camera image data to determine information on an angular orientation of the head of the user and to determine a fixation of the user on a point. The method further comprises providing 330 a control signal for a robotic adjustment system of the microscope system based on the information on the angular orientation of the head of the user such that a central point of view of a microscope of the microscope system remains fixed on the point.

As indicated above, features described in connection with the apparatus 110 and the microscope system 100 of Figs. 1a and/or 1b may be likewise applied to the method of Fig. 3.

More details and aspects of the method are mentioned in connection with the proposed concept or one or more examples described above or below (e.g. Fig. 1, 2 or 4). The method may comprise one or more additional optional features corresponding to one or more aspects of the proposed concept or one or more examples described above or below.

At least some embodiments relate to the use of an imaging system that is attached to a robotic arm. The use of an imaging system (e.g. surgical microscope) attached on a robotic arm, which moves the imaging system in space (typically 6 degrees of freedom) allows many adjustments of the imaging settings, such as:
1. Position in space, *x,y,z* (3 degrees of freedom)
2. Observation angle *θ,φ,ω* (3 degrees of freedom)
3. Zoom, working distance, illumination/sensitivity (3 degrees of freedom)

Such adjustment may be complex when performed with conventional knob/button adjustments. Moreover, such adjustments may require time and the attention of the user. Moreover, it may be demanding to perform complex adjustments such as point lock (fix the observation center, while changing the observation angle), which may require a combined adjustment of parameters (e.g. positioning lies on a sphere, while observation angle adapts accordingly).

Human vision, however, is mechanically/geometrically similar to the robotic imaging system, and humans intuitively adjust the observation geometry in order to perceive the shape of an object. Embodiments provide a control system (e.g. the apparatus 110 of Figs. 1a and/or 1b) which allows humans to control a robotic imaging system (e.g. the microscope system 100 of Figs. 1a and/or 1b) in an intuitive way which resembles the natural observation motions/gestures. In particular, embodiments may focus on the recognition of the human's posture and movement of head (e.g. the angular orientation of the head) and interpret it as a guide for the arrangement of the robotic imaging system (e.g. by providing the control signal). Such a system could be any combination of the following concepts:
1. Replication of the human head observation geometry, i.e. head position (x,y,z) and angle (*θ,φ,ω*) (i.e. the angular orientation). In addition, also eye-tracking information may be included.
2. Interpretation of the movements/gestures of the human head in order to perform certain adjustments. For example, when head is moving in space (x,y,z) without turning then it could mean pan of the field of view, while when (x,y,z) movement is combined with counterbalancing turning of the head (*θ,φ,ω*), then it may be interpreted as point-lock adjustment. In addition, eye-tracking and shoulder position could also be used for a more robust interaction.

More details and aspects of the concept are mentioned in connection with the proposed concept or one or more examples described above or below (e.g. Fig. 1 to 3). The concept may comprise one or more additional optional features corresponding to one or more aspects of the proposed concept or one or more examples described above or below.

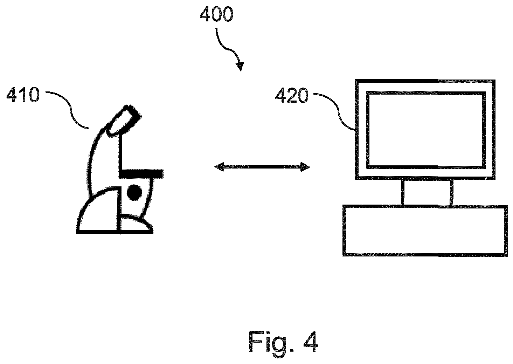

Some embodiments relate to a microscope comprising a system as described in connection with one or more of the Figs. 1 to 3. Alternatively, a microscope may be part of or connected to a system as described in connection with one or more of the Figs. 1 to 3. Fig. 4 shows a schematic illustration of a system 400 configured to perform a method described herein. The system 400 comprises a microscope 410, which may correspond to the microscope 150 of Fig. 1b, and a computer system 420, which may correspond to the apparatus 110 of Figs. 1a and/or 1b. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference Signs

- 100: Microscope system
- 110: Apparatus for controlling a microscope system
- 112: Interface
- 114: Processing module
- 120: Camera module
- 130: Robotic adjustment system
- 140: Display (suffixes a-c)
- 145: Arm
- 150: Microscope
- 155: Arm
- 160: Input device
- 210: Obtaining camera image data
- 220: Processing the camera image data
- 230: Providing a control signal
- 310: Obtaining camera image data
- 320: Processing the camera image data
- 330: Providing a control signal
- 400: System
- 410: Microscope
- 420: Computer system

## Claims

1. An apparatus (110; 420) for controlling a microscope system (100; 400), the apparatus comprising:
an interface (112) for communicating with a camera module (120), the camera module being suitable for providing camera image data of a head of a user of the microscope system;
a processing module (114) configured to:
obtain the camera image data from the camera module via the interface,
process the camera image data to determine information on an angular orientation of the head of the user relative to a display (140) of the microscope system by determining the angle of view of the user towards the display, and
provide a control signal for a robotic adjustment system (130) of the microscope system based on the information on the angular orientation of the head of the user, wherein the control signal is provided such, that the robotic adjustment system is affected to arrange the microscope (150) at an angle that is based on the angle of view of the user.

2. The apparatus according to claim 1, wherein the processing module is configured to determine an angle of view of the user of the microscope system towards the display based on the camera image data, and to provide the control signal based on the angle of view of the user.

3. The apparatus according to claim 2, wherein the processing module is configured to determine the angle of view of the user based on the angular orientation of the head of the user.

4. The apparatus according to claim 3, wherein the processing module is configured to perform eye tracking on one or both eyes of the user, and to determine the angle of view of the user based on the angular orientation of the head of the user and based on the eye tracking.

5. The apparatus according to one of the claims 1 to 4, wherein the processing module is configured to determine the information on the angular orientation using image analysis on the camera image data.

6. The apparatus according to one of the claims 1 to 5, wherein the processing module is configured to process the camera image data to deter-mine the information on the angular orientation of the head of the user relative to an auxiliary display that is arranged in proximity of an eyepiece of a microscope of the microscope system,
or wherein the processing module is configured to process the camera image data to deter-mine the information on the angular orientation of the head of the user relative to an auxiliary display that is arranged at a base unit of the microscope system,
or wherein the processing module is configured to process the camera image data to deter-mine the information on the angular orientation of the head of the user relative to an ocular display of an eyepiece of a microscope of the microscope system.

7. The apparatus according to one of the claims 1 to 6, wherein the processing module is configured to determine a fixation of the user on a point based on the camera image data, wherein the processing module is configured to determine the control signal for the robotic adjustment system such that a central point of view of a microscope (150; 410) of the microscope system remains fixed on the point.

8. The apparatus according to one of the claims 1 to 7, wherein the processing module is configured to determine information on a position of the head of the user and information on an angle of the head of the user based on the camera image, and to determine the information on the angular orientation of the head of the user based on the information on the position of the head of the user and based on the information on the angle of the head of the user.

9. The apparatus according to claim 8, wherein the processing module is configured to generate the control signal such that the robotic adjustment system is triggered to pan a field of view of a microscope of the microscope system if the position of the head of the user changes without a corresponding change in the angle of the head of the user,
and/or wherein the processing module is configured to generate the control signal such that the robotic adjustment system is triggered to adjust an angle of view of a microscope of the microscope system if the position of the head of the user changes with a corresponding change in the angle of the head of the user.

10. The apparatus according to one of the claims 1 to 9, wherein the processing module is configured to determine a fixation of the user on a point, and
to provide the control signal for the robotic adjustment system (130) of the microscope system such that a point of view of a microscope (150; 410) of the microscope system remains fixed on the point.

11. A method for controlling a microscope system, the method comprising:
obtaining (210) camera image data of a head of a user of a head of a user of the microscope system from a camera module of the microscope system;
processing (220) the camera image data to determine information on an angular orientation of the head of the user relative to a display of the microscope system by determining the angle of view of the user towards the display; and
providing (230) a control signal for a robotic adjustment system of the microscope system based on the information on the angular orientation of the head of the user, wherein the control signal is provided such, that the robotic adjustment system is affected to arrange the microscope (150) at an angle that is based on the angle of view of the user.

12. The method for controlling a microscope according to claim 11, wherein
processing (320) the camera image data to determine information on an angular orientation of the head of the user relative to a display of the microscope by determining the angle of view of the user towards the display further comprises determining a fixation of
the user on a point, and wherein providing (330) a control signal for a robotic adjustment system of the microscope system based on the information on the angular orientation of the head of the user such that the robotic adjustment system is affected to arrange the microscope (150) at an angle that is based on the angle of view of the user is carried out such that a point of view of a microscope (150) of the microscope system remains fixed on the point.

13. A computer program with a program code for performing at least one of the methods
according to one of the claims 11 or 12 when the computer program is executed on the processing module of claim 1.

## Patentansprüche

1. Vorrichtung (110; 420) zur Steuerung eines Mikroskopsystems (100; 400), die Vorrichtung umfassend:
eine Schnittstelle (112) zur Kommunikation mit einem Kameramodul (120), wobei das Kameramodul dazu eingerichtet ist, Kamerabilddaten eines Kopfes eines Benutzers des Mikroskopsystems bereitzustellen;
ein Verarbeitungsmodul (114), das konfiguriert ist zum:
Erhalten der Kamerabilddaten von dem Kameramodul über die Schnittstelle, Verarbeiten der Kamerabilddaten, um Informationen über eine Winkelorientierung des Kopfes des Benutzers relativ zu einer Anzeige (140) des Mikroskopsystems durch Bestimmen des Blickwinkels des Benutzers zu der Anzeige hin zu bestimmen, und
Bereitstellen eines Steuersignals für ein robotisches Einstellsystem (130) des Mikroskopsystems basierend auf den Informationen über die Winkelorientierung des Kopfes des Benutzers, wobei das Steuersignal derart bereitgestellt wird, dass das robotische Einstellsystem veranlasst wird, das Mikroskop (150) in einem Winkel anzuordnen, der auf dem Blickwinkel des Benutzers basiert.

2. Vorrichtung nach Anspruch 1, wobei das Verarbeitungsmodul dazu konfiguriert ist, einen Blickwinkel des Benutzers des Mikroskopsystems zu der Anzeige hin basierend auf den Kamerabilddaten zu bestimmen und das Steuersignal basierend auf dem Blickwinkel des Benutzers bereitzustellen.

3. Vorrichtung nach Anspruch 2, wobei das Verarbeitungsmodul dazu konfiguriert ist, den Blickwinkel des Benutzers basierend auf der Winkelorientierung des Kopfes des Benutzers zu bestimmen.

4. Vorrichtung nach Anspruch 3, wobei das Verarbeitungsmodul dazu konfiguriert ist, eine Augenverfolgung an einem oder beiden Augen des Benutzers durchzuführen und den Blickwinkel des Benutzers basierend auf der Winkelorientierung des Kopfes des Benutzers und basierend auf der Augenverfolgung zu bestimmen.

5. Vorrichtung nach einem der Ansprüche 1 bis 4, wobei das Verarbeitungsmodul dazu konfiguriert ist, die Informationen über die Winkelorientierung unter Verwendung einer Bildanalyse an den Kamerabilddaten zu bestimmen.

6. Vorrichtung nach einem der Ansprüche 1 bis 5, wobei das Verarbeitungsmodul dazu konfiguriert ist, die Kamerabilddaten zu verarbeiten, um die Informationen über die Winkelorientierung des Kopfes des Benutzers relativ zu einer Hilfsanzeige zu bestimmen, die in der Nähe eines Okulars eines Mikroskops des Mikroskopsystems angeordnet ist, oder wobei das Verarbeitungsmodul dazu konfiguriert ist, die Kamerabilddaten zu verarbeiten, um die Informationen über die Winkelorientierung des Kopfes des Benutzers relativ zu einer Hilfsanzeige zu bestimmen, die an einer Basiseinheit des Mikroskopsystems angeordnet ist, oder wobei das Verarbeitungsmodul dazu konfiguriert ist, die Kamerabilddaten zu verarbeiten, um die Informationen über die Winkelorientierung des Kopfes des Benutzers relativ zu einer Okularanzeige eines Okulars eines Mikroskops des Mikroskopsystems zu bestimmen.

7. Vorrichtung nach einem der Ansprüche 1 bis 6, wobei das Verarbeitungsmodul dazu konfiguriert ist, basierend auf den Kamerabilddaten eine Fixierung des Benutzers auf einen Punkt zu bestimmen, wobei das Verarbeitungsmodul dazu konfiguriert ist, das Steuersignal für das robotische Einstellsystem derart zu bestimmen, dass ein zentraler Blickpunkt eines Mikroskops (150; 410) des Mikroskopsystems auf den Punkt fixiert bleibt.

8. Vorrichtung nach einem der Ansprüche 1 bis 7, wobei das Verarbeitungsmodul dazu konfiguriert ist, basierend auf dem Kamerabild Informationen über die Position des Kopfes des Benutzers und Informationen über einen Winkel des Kopfes des Benutzers zu bestimmen und basierend auf den Informationen über die Position des Kopfes des Benutzers und basierend auf den Informationen über den Winkel des Kopfes des Benutzers die Informationen über die Winkelorientierung des Kopfes des Benutzers zu bestimmen.

9. Vorrichtung nach Anspruch 8, wobei das Verarbeitungsmodul dazu konfiguriert ist, das Steuersignal derart zu erzeugen, dass das robotische Einstellsystem ausgelöst wird, um ein Sichtfeld eines Mikroskops des Mikroskopsystems zu schwenken, falls sich die Position des Kopfes des Benutzers ohne eine entsprechende Änderung des Winkels des Kopfes des Benutzers ändert, und/oder wobei das Verarbeitungsmodul dazu konfiguriert ist, das Steuersignal derart zu erzeugen, dass das robotische Einstellsystem ausgelöst wird, um den Blickwinkel eines Mikroskops des Mikroskopsystems einzustellen, falls sich die Position des Kopfes des Benutzers mit einer entsprechenden Änderung des Winkel des Kopfes des Benutzers ändert.

10. Vorrichtung nach einem der Ansprüche 1 bis 9, wobei das Verarbeitungsmodul dazu konfiguriert ist, eine Fixierung des Benutzers auf einen Punkt zu bestimmen, und das Steuersignal für das robotische Einstellsystem (130) des Mikroskopsystems derart bereitzustellen, dass ein Blickpunkt eines Mikroskops (150; 410) des Mikroskopsystems auf den Punkt fixiert bleibt.

11. Verfahren zur Steuerung eines Mikroskopsystems, das Verfahren umfassend:
Erhalten (210) von Kamerabilddaten eines Kopfes eines Benutzers des Mikroskopsystems von einem Kameramodul des Mikroskopsystems;
Verarbeiten (220) der Kamerabilddaten, um Informationen über eine Winkelorientierung des Kopfes des Benutzers relativ zu einer Anzeige des Mikroskopsystems durch Bestimmen des Blickwinkels des Benutzers zu der Anzeige hin zu bestimmen; und
Bereitstellen (230) eines Steuersignals für ein robotisches Einstellsystem des Mikroskopsystems basierend auf den Informationen über die Winkelorientierung des Kopfes des Benutzers, wobei das Steuersignal derart bereitgestellt wird, dass das robotische Einstellsystem veranlasst wird, das Mikroskop (150) in einem Winkel anzuordnen, der auf dem Blickwinkel des Benutzers basiert.

12. Verfahren zur Steuerung eines Mikroskops nach Anspruch 11, wobei das Verarbeiten (320) der Kamerabilddaten, um Informationen über eine Winkelorientierung des Blickwinkels des Benutzers zu der Anzeige hin zu bestimmen, weiter Bestimmen einer Fixierung des Benutzers auf einen Punkt umfasst, und wobei das Bereitstellen (330) eines Steuersignals für ein robotisches Einstellsystem des Mikroskopsystems basierend auf den Informationen über die Winkelorientierung des Kopfes des Benutzers, derart, dass das robotische Einstellsystem veranlasst wird, das Mikroskop (150) in einem Winkel anzuordnen, der auf dem Blickwinkel des Benutzers basiert, derart ausgeführt wird, dass ein Blickpunkt eines Mikroskops (150) des Mikroskopsystems auf den Punkt fixiert bleibt.

13. Computerprogramm mit einem Programmcode, der dazu eingerichtet ist, mindestens eines der Verfahren nach einem der Ansprüche 11 oder 12 durchzuführen, wenn das Computerprogramm auf dem Verarbeitungsmodul nach Anspruch 1 ausgeführt wird.

## Revendications

1. Appareil (110 ; 420) destiné à commander un système de microscope (100 ; 400), l'appareil comprenant :
une interface (112) destinée à communiquer avec un module de caméra (120), le module de caméra étant approprié pour fournir des données d'image de caméra d'une tête d'un utilisateur du système de microscope ;
un module de traitement (114) configuré pour :
obtenir les données d'image de caméra à partir du module de caméra par le biais de l'interface, traiter les données d'image de caméra afin de déterminer des informations relatives à une orientation angulaire de la tête de l'utilisateur par rapport à un écran (140) du système de microscope en déterminant l'angle de vision de l'utilisateur vers l'écran, et
fournir un signal de commande pour un système de réglage robotisé (130) du système de microscope sur la base des informations relatives à l'orientation angulaire de la tête de l'utilisateur, dans lequel le signal de commande est fourni de telle sorte que le système de réglage robotisé soit affecté pour agencer le microscope (150) selon un angle qui est basé sur l'angle de vision de l'utilisateur.

2. Appareil selon la revendication 1, dans lequel le module de traitement est configuré pour déterminer un angle de vision de l'utilisateur du système de microscope vers l'écran sur la base des données d'image de caméra, et pour fournir le signal de commande sur la base de l'angle de vision de l'utilisateur.

3. Appareil selon la revendication 2, dans lequel le module de traitement est configuré pour déterminer l'angle de vision de l'utilisateur sur la base de l'orientation angulaire de la tête de l'utilisateur.

4. Appareil selon la revendication 3, dans lequel le module de traitement est configuré pour effectuer une poursuite oculaire sur l'un ou les deux yeux de l'utilisateur et pour déterminer l'angle de vision de l'utilisateur sur la base de l'orientation angulaire de la tête de l'utilisateur et sur la base de la poursuite oculaire.

5. Appareil selon l'une des revendications 1 à 4, dans lequel le module de traitement est configuré pour déterminer les informations relatives à l'orientation angulaire en utilisant l'analyse d'images sur les données d'image de caméra.

6. Appareil selon l'une des revendications 1 à 5, dans lequel le module de traitement est configuré pour traiter les données d'image de caméra afin de déterminer les informations relatives à l'orientation angulaire de la tête de l'utilisateur par rapport à un écran auxiliaire qui est agencé à proximité d'un oculaire d'un microscope du système de microscope, ou dans lequel
le module de traitement est configuré pour
traiter les données d'image de caméra afin de déterminer les informations relatives à l'orientation angulaire de la tête de l'utilisateur par rapport à un écran auxiliaire qui est agencé au niveau d'une unité de base du système de microscope, ou dans lequel le module de traitement est configuré pour traiter les données d'image de caméra afin de déterminer les informations relatives à l'orientation angulaire de la tête de l'utilisateur par rapport à un écran oculaire d'un oculaire d'un microscope du système de microscope.

7. Appareil selon l'une des revendications 1 à 6, dans lequel le module de traitement est configuré pour déterminer une fixation de l'utilisateur sur un point sur la base des données d'image de caméra, dans lequel le module de traitement est configuré pour déterminer le signal de commande pour le système de réglage robotisé de telle sorte qu'un point de vision central d'un microscope (150 ; 410) du système de microscope reste fixé sur le point.

8. Appareil selon l'une des revendications 1 à 7, dans lequel le module de traitement est configuré pour déterminer des informations relatives à une position de la tête de l'utilisateur et des informations relatives à un angle de la tête de l'utilisateur sur la base de l'image de caméra et pour déterminer les informations relatives à l'orientation angulaire de la tête de l'utilisateur sur la base des informations relatives à la position de la tête de l'utilisateur et sur la base des informations relatives à l'angle de la tête de l'utilisateur.

9. Appareil selon la revendication 8, dans lequel le module de traitement est configuré pour générer le signal de commande de telle sorte que le système de réglage robotisé soit déclenché pour faire pivoter un champ de vision d'un microscope du système de microscope si la position de la tête de l'utilisateur change sans un changement correspondant de l'angle de la tête de l'utilisateur,
et/ou dans lequel le module de traitement est configuré pour générer le signal de commande de telle sorte que le système de réglage robotisé soit déclenché pour régler un angle de vision d'un microscope du système de microscope si la position de la tête de l'utilisateur change avec un changement correspondant de l'angle de la tête de l'utilisateur.

10. Appareil selon l'une des revendications 1 à 9, dans lequel le module de traitement est configuré pour déterminer une fixation de l'utilisateur sur un point et
pour fournir le signal de commande pour le système de réglage robotisé (130) du système de microscope de telle sorte qu'un point de vision d'un microscope (150 ; 410) du système de microscope reste fixé sur le point.

11. Procédé destiné à commander un système de microscope, le procédé comprenant :
obtenir (210) des données d'image de caméra d'une tête d'un utilisateur d'une tête d'un utilisateur du système de microscope à partir d'un module de caméra du système de microscope ;
traiter (220) les données d'image de caméra afin de déterminer des informations relatives à une orientation angulaire de la tête de l'utilisateur par rapport à un écran du système de microscope en déterminant l'angle de vision de l'utilisateur vers l'écran ; et
fournir (230) un signal de commande pour un système de réglage robotisé du système de microscope sur la base des informations relatives à l'orientation angulaire de la tête de l'utilisateur, dans lequel le signal de commande est fourni de telle sorte que le système de réglage robotisé soit affecté pour agencer le microscope (150) selon un angle qui est basé sur l'angle de vision de l'utilisateur.

12. Procédé destiné à commander un microscope selon la revendication 11, dans lequel le fait de traiter (320) des données d'image de caméra afin de déterminer des informations relatives à une orientation angulaire de la tête de l'utilisateur par rapport à un écran du microscope en déterminant l'angle de vision de l'utilisateur vers l'écran comprend en outre le fait de déterminer une fixation de l'utilisateur sur un point, et dans lequel le fait de fournir (330) un signal de commande pour un système de réglage robotisé du système de microscope sur la base des informations relatives à l'orientation angulaire de la tête de l'utilisateur de telle sorte que le système de réglage robotisé soit affecté pour agencer le microscope (150) selon un angle qui est basé sur l'angle de vision de l'utilisateur est réalisé de telle sorte qu'un point de vision du microscope (150) du système de microscope reste fixé sur le point.

13. Programme d'ordinateur avec un code de programme destiné à effectuer au moins l'un des procédés selon l'une des revendications 11 ou 12 lorsque le programme d'ordinateur est exécuté sur le module de traitement selon la revendication 1.
